(19) Europäisches Patentamt
European Patent Office
Office européen des brevets

(11) **EP 4 388 978 A1**

(12) **EUROPEAN PATENT APPLICATION**

(43) Date of publication:
**26.06.2024 Bulletin 2024/26**

(21) Application number: **22461644.1**

(22) Date of filing: **19.12.2022**

(51) International Patent Classification (IPC):
**A61B 3/11** (2006.01)　　**G06F 21/32** (2013.01)
**G06V 40/19** (2022.01)　　**G02C 13/00** (2006.01)

(52) Cooperative Patent Classification (CPC):
**A61B 3/112; G06F 21/32; G06V 40/19;**
G02C 13/005

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Solvemed Group sp. z o.o.
61-806 Poznan (PL)**

(72) Inventors:
• **CHRAPKIEWICZ, Radoslaw
Sunnyvale, CA 94087 (US)**
• **JACHURA, Michal
02-353 Warszawa (PL)**
• **WLODARSKI, Michal
85-829 Bydgoszcz (PL)**
• **CHROST, Hugo
64-100 Leszno (PL)**

(74) Representative: **AOMB Polska Sp. z.o.o.
ul. Rondo Ignacego Daszynskiego 1
00-843 Warsaw (PL)**

(54) **METHOD AND SYSTEM FOR ABSOLUTE MEASUREMENT OF DIAMETER OF A PART OF AN EYE**

(57) The subject matter of the present invention is method for absolute measurement of diameter of a part of an eye, using a visible light camera with adjustable focus and a system configured and programmed for performing said method.

Fig. 4

**Description**

**Technical field**

The present disclosure relates to the field of medicine, ophthalmology, neuroophthalmology with a subfield of pupillometry specializing in absolute determination of a pupil and/or iris size using dynamic assessment reflecting the subject medical condition. The subject matter of the invention is a method for the dynamic absolute measurement of a pupil and/or iris diameter relying on the plurality of images recorded by any camera equipped with an adjustable focus for the purpose of pupillometric measurements.

Prior art

In the state of the art, a variety of pupil measurement systems and methods are reported. For instance, patent document published in 2018 [WO2018222897A1] proposes a technique employing machine learning to enable smartphone-based visible light pupillometry. A smartphone, that is a device which might be used to record a visible light video of a pupillary light reflex (PLR) and afterwards a machine learning model might be used to retrieve a size of a pupil along with its diameter change over time. In contrast to the proposed method, the solution requires a specially designed box which simultaneously reduces ambient illumination of the eye and controls the distance between the subject's face and the camera so that pixel sizes may be converted into absolute measurement sizes, i.e. expressed in physical units such as inches or millimetres. In some embodiments instead of the aforementioned box the authors propose that the subject will hold an object of known size during the measurement, such as an ID card, drivers' license, coin, etc., next to the eye in order to translate the pupil image pixel size into actual dimensions.

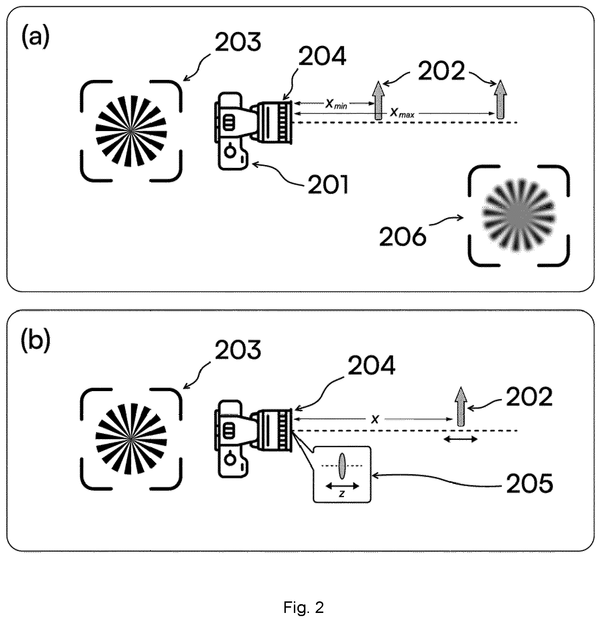

Another patent document published in 2017 [US10034605B2] describes systems, devices, and methods that may be used to detect and determine the extent of brain trauma, mental impairment, physical disability, or other brain dysfunction by tracking pupillary light response. Although the picture in the document (Fig. 2) suggests that the system is able to measure pupil diameter expressed in absolute values (millimetres), the document does not teach how this type of the measurement is performed. Moreover, in the patent claims only the relative size of patient's pupil is mentioned, which is afterwards used for calculating pupillometric parameters such as the rates of change of the pupil size or the deviation of thereof.

In patent document [WO2014163891A1] a system for analysing the anatomy of a patient's eye with circular or rotated polarized laser beams, or with laser beams of different wavelengths is disclosed. The analysis is based on the back-reflected laser beam from the eye. The anatomical features including pupil diameter, cornea curvature, lens, capsular bag etc. are retrieved for the purpose of laser eye surgery. The system operates in the contact regime i.e., the objective component with a focusing lens directly contacts the eye surface which limits it's use to highly controlled clinical environment. Using the pulsed laser beam also pose a series of eye-safety and regulatory issues and dramatically increases the cost of the device.

Patent document [WO2020168009A1] reveals a method for examining a response from one or more pupils of an eye. The method is based on hand-held mobile device with a display, a built-in camera, and a light stimulus source, wherein the light stimulus source can emit light at various intensities. The method uses mobile device that is used to locate the pupil, to determine a distance between the device and the pupil and to apply an algorithm that calculates a specific or optimal light intensity based on the distance between the device and the eye and causes the light stimulus source to emit one or more flashes of light at the specific or optimal light intensity. Moreover, the built-in camera of hand-held mobile device is used to record the response of the pupil to the one or more flashes of light; and displaying on the display the data representative of the response of the pupil to the one or more flashes of light. In order to provide an estimate for the pupil diameter the inventors propose to first measure the diameter of the iris of the eye (by measuring the sclera/iris border), assuming that the iris diameter equals the population average and finally comparing the diameter of the pupil to the diameter of an iris.

Patent document [WO2021211886A1] describes a modular platform, containing harness, that is configured to fit a head of a patient. Patent document describes a device for a screening platform that enables comprehensive ocular evaluations. The screening platform includes a harness that is configured to fit a head of a patient and that includes one or more electronic components that are operable to power an interchangeable module, a central processing unit (CPU) with a graphical processing unit (GPU), and the interchangeable module with communication capabilities to external computational devices, multiple display output devices, and several types of input devices from both an operator and a patient at-hand, wherein the interchangeable module is separable from the screening platform. Although among interchangeable modules the module for pupillary diameter measurements is proposed, the inventors do not specify how this type of a measurement is planned to be realized.

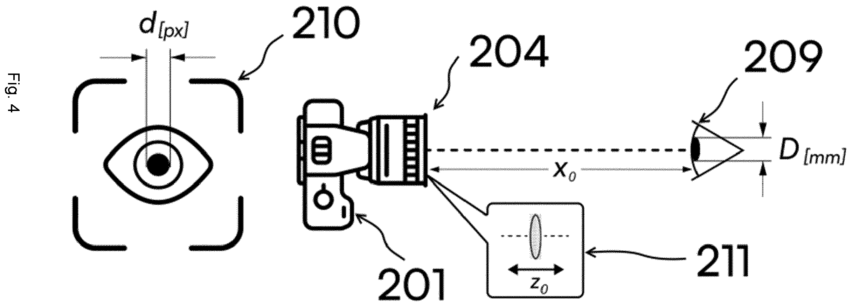

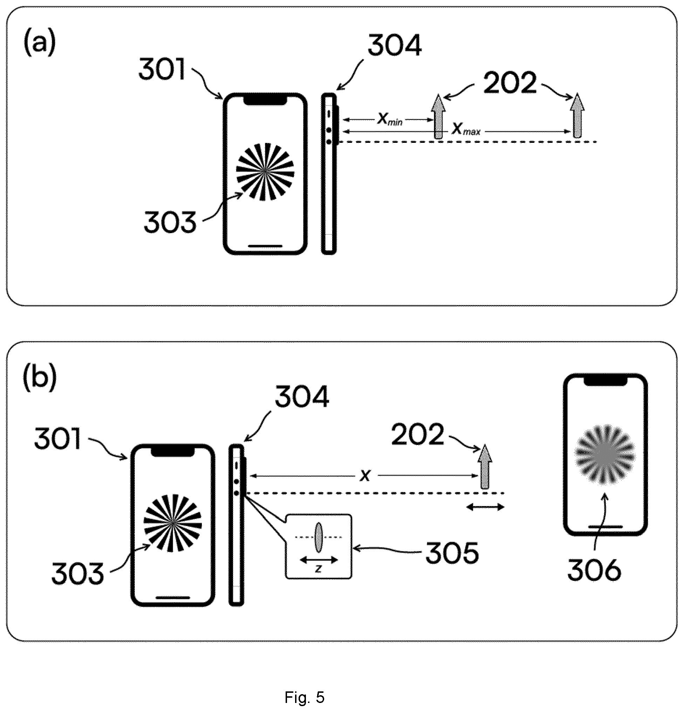

Patent document [US11122972B2] describes a method for pupil size measurement with a closed eyelid of a subject which relies on transcranial transmission of light. The device and method allow for the determination of a pupil size in a subject having closed eyelids. It requires a specially designed eyeglasses presented in Fig. 4 and Fig. 5A, 5B of the document which are equipped with light detector arrays. According to some embodiment the method relies on ultrasound transducer configured to provide ultrasound outputs of a subject's eye. According to other embodiments the light detectors may include an IR camera, sensitive to near-IR, configured to enable detection of the pupil contour and/or pupil shape by near-IR imaging.

US patent document published in 2016 [US9720259B2] describes an eyewear pupilometer, that is an independent apparatus including an eyewear pupilometer for detecting, measuring and processing a wearer's pupil movement and size, for detecting and processing retinal images and for detecting and processing a wearer's field of view. The apparatus is designed for broadcasting alerts, for pre-diagnostic screening, for overriding vehicle operation and for measuring, recording and transmitting circadian responses. An eyewear pupilometer module could be embedded into eyewear frames and lenses, contact lenses or a vehicle wind shield. The eyewear pupilometer module comprises a lens fibre optic camera module, an image processor module, a retinal image infrared detector module and an outward view camera module. The device uses infrared image detection for determining if the pupil is dilated or not.

Another prior art document published in 2017 [US20170347878A1] disclosed a method and system for monitoring and/or assessing pupillary responses. It describes a method for monitoring and/or assessing pupillary responses to continuous illumination of light starting at a first intensity which is gradually changed to subsequent intensity over a predefined period of time. The method may be used to detect aberrant pupillary responses - for instance diabetes are to cause eye dysfunction such as diabetic retinopathy. The embodiments of the system implementing aforementioned method is presented in Fig. 11 and Fig. 12. Both embodiments comprise infrared light source and the infrared camera suitably positioned to capture the images of the eye. They are also equipped with an eye-piece with a protective cover which blocks the external illumination and controls the distance between the subject's eye and the camera.

Problem from the state of the art

In the prior art there exists pervasive problem of the precise absolute measurement of the human eye pupil and/or iris size without the need of controlling the distance between eye and a mobile camera device as well as without the need of using auxiliary physical objects of a well-known size during the measurement. The indicated problem of the precise pupil and/or iris diameter absolute measurement has been tackled in many ways requiring auxiliary physical objects or statistical assumptions such as:

(i) controlling a distance between eye and a mobile imaging device camera - by the means of a rubber cap, an eyewear or other distance-fixing tool;

(ii) usage of a coin, credit card or any other physical object of a well-known size to be placed during the measurement in a field of view near a subject's eye for the dimensions reference;

(iii) assuming that the actual iris diameter equals the statistical population average and calculating the pupil and/or iris diameter based on the aforementioned size of the iris;

(iv) a need for an infrared emitter and detector to increase the pupil/iris contrast and reduce the detrimental effect of visible light eye reflections.

The problem is also the usage of the unknown (e.g., due to secret know-how or patent protection) mobile camera with the adjustable focus for the purpose of precise pupillometric measurements. Thus, there exists a need for affordable and reliable method for measurements of the human eye pupil and/or iris diameter without the pre-existing knowledge about parameters such as: (i) distance between the eye and a mobile camera; (ii) magnification of the optical imaging system in the camera; (iii) pixel size of the light sensor in the camera; (iv) the relation between focusing element position and the focusing distance.

**Solution to the problem**

The invention described in method relies on performing a series of calibration steps of the unknown visible light mobile camera with adjustable focus. In the first step the focusing distance is calibrated against the position of the focusing element inside the camera. Extreme positions of focusing element define the maximal and minimal focusing distances. Afterwards, the image scaling of the system is measured either for expected eye-to-camera distance range or for the entire operating range of focusing distance. Once the calibration is done, the eye-to-camera distance is inferred from the position of the focusing element and the pupil/iris image diameter measured by the light sensor in pixels is translated into the actual diameter by the means of previously retrieved image scaling relation. This allows for a reliable absolute measurement of a pupil and/or iris diameter without a pre-existing knowledge about optical imaging system in the camera and without any additional tool for fixing the eye-to-camera distance. The method allows for broad usage of mobile visible light cameras for the purpose of pupillometry, as well as biometric identification, eye socket/orbit surgery etc.

**Glossary and definitions of terms known in the state of the art and used in the patent specification**

Throughout the present specification (both above and below the glossary section), the terms used have the following meanings described in the definitions below:
The term "dynamic measurement" means that the measurements are based on the plurality of images and thus might be used to recover changes of measured values over time. In particular, when a plurality of images are collected for a specific distance between the camera and the eye $x_0$ then a plurality of pupil/iris diameters are obtained, which correspond to changes of said diameter over time.

The term "light sensor" means a CCD image sensor, EMCCD image sensor, CMOS image sensor, sCMOS sensor or other optoelectronic device containing an array of light sensitive pixels.

The term "scaling object" means any object equipped with dimension scale such as a ruler, graph paper or tape measure as well as any object which size is known such as a specific coin, ID card or matchbox.

The term "(mobile) camera with adjustable focus" means electronic device equipped with a light sensor and optical imaging system with adjustable focusing distance, which can capture an image. This includes a webcam, a video camera, a digital camera, portable video device, or a smartphone equipped with one or several cameras. The said optical imaging system with adjustable focusing distance contains a plurality of lenses.

The term "absolute measurement" means that the measurement result is expressed in physical units such as millimetres [mm] or inches [in].

The term "optical target" means any object with recognizable spatial features such as checkerboard, Ronchi ruling, Siemens star or specially dedicated targets such as 1951 USAF test chart. The spatial features of the object have to be diverse enough to ensure efficient working of the focusing algorithm in the camera.

The term "focusing element" means a moveable plurality of lenses (or lens assembly) or a single lens which is a part of camera imaging system and is responsible for setting the focusing distance of the camera.

The term "image scaling" denotes a relation between the actual size of an object and its focused image size on the light sensor. The scaling depends on the position of the object along camera optical axis (focusing distance) and can be expressed e.g., in millimetres per pixel.

The term "focusing distance" means a distance from the camera to the object plane, which focused image is formed on the light sensor by the means of camera imaging system.

The term "unknown camera/imaging system/lenses" refers to an optical system, in particular a camera or at least an arrangement of optical lenses, the characteristics of which, including at least maximal and minimal focusing distance, the relation between focusing element position and focusing distance as well as magnification is not known in advance.

In the present document, other terms not defined above have meanings which are given and understood by a professional in the field in light of the best knowledge available, the present disclosure and the context of the patent specification.

**Summary of the invention**

A method for absolute measurement of diameter of a part of an eye, using a visible light camera with adjustable focus, said camera having the following camera characteristics:

- the minimal and maximal focusing camera-to-object distance $x_{min}$ and $x_{max}$ of the camera imaging system;
- the $z(x)$ relation, for $x \in [x_{min}, x_{max}]$, wherein $z(x)$ is the position of the focusing element of the camera which results in a focused image on an object located at the distance $x$ from the camera;
- the $H/h(x)$ relation, for $x \in [x_{min}, x_{max}]$, wherein $H/h(x)$ is the image scaling ratio of the size $H$ of a physical object, placed at the distance $x$ from the camera, to the size $h$ of its focused image captured by the camera

according to the invention comprises the following steps:

(m1) directing the camera towards the subject's eye and recording a single focused image or a plurality of focused images of the part of the subject's eye, while reading out corresponding focusing element position(s) $z_0$ and measuring corresponding diameter(s) $d$ of the focused image(s) of the part of the eye;
(m2) calculating corresponding camera-to-eye distance(s) $x_0$ based on the focusing element position(s) $z_0$ using the inverse of the $z(x)$ relation:

$$x_0 = z^{-1}(z_0) \qquad (1)$$

(m3) converting the diameter(s) $d$ of one or more images of the part of the eye obtained in step (m1) into actual diameter(s) $D$ of the part of the eye for each focused image collected in step (m1), using the $H/h(x)$ relation and the corresponding value(s) of $x_0$ obtained in step (m2):

$$D = d * H/h(x_0) \qquad (2)$$

In one option, said camera characteristics is provided by a third party, such as the camera's manufacturer, preferably in the form of an interpolated curve, a fitted analytical curve or a lookup table.

In another option, said camera characteristics is established in a procedure comprising the following steps:

(c1) establishing the minimal and maximal focusing camera-to-object distance $x_{min}$ and $x_{max}$ of the camera imaging system;

(c2) reading out focusing element position $z(x)$ resulting in a focused image of an optical target located at the distance $x$ from the camera, for at least some $x \in [x_{min}, x_{max}]$, preferably for the entire operating range of the focusing distance $[x_{min}, x_{max}]$, by means of the optical target moved along the optical axis of the camera, thereby obtaining the $z(x)$ relation for $x \in [x_{min}, x_{max}]$;

(c3) measuring focused image scaling ratio $H/h(x)$ of the physical size $H$ of a scaling object, placed at the distance $x$ from the camera, to the size h of its focused image obtained in the camera, for at least some $x \in [x_{min,} x_{max}]$, preferably for the entire operating range of the focusing distance $[x_{min}, x_{max}]$, by means of the scaling object moved along the optical axis of the camera, thereby obtaining the $H/h(x)$ relation for $x \in [x_{min}, x_{max}]$.

In such case, preferably, in steps (c1) and/or (c2) the focusing element position resulting in the focused image is set using a built-in algorithm implemented in the camera.

Alternatively, in steps (c1) and/or (c2) the focusing element position resulting in the focused image is set manually by a user.

Preferably, the camera is a camera with adjustable focus being a part of a mobile electronic device, in particular is a built-in camera of a smartphone or a tablet

In such case, the mobile electronic device may contain a single camera or a plurality of cameras.

Preferably, the camera is a camera with adjustable focus which contains a plurality of lenses.

Preferably, the camera contains an imaging system with f-number in a range of f/0.95-f/2.8.

Preferably, the distance between camera imaging system and the subject's eye is more than 1.5 cm and less than 75 cm, preferably more than 2 cm and less than 50 cm, more preferably more than 3 cm and less than 40 cm, still more preferably more than 4 cm and less than 30 cm.

Preferably, the part of an eye is a pupil or an iris.

Preferably, the method is computer-implemented.

The invention includes also a method for absolute measurement of a distance between a visible light camera and a subject's eye, wherein the camera has adjustable focus and the following camera characteristics:

- the minimal and maximal focusing camera-to-object distance $x_{min}$ and $x_{max}$ of the camera imaging system;
- the $z(x)$ relation, for $x \in [x_{min}, x_{max}]$, wherein $z(x)$ is the position of the focusing element of the camera which results in a focused image on an object located at the distance $x$ from the camera;
- the $H/h(x)$ relation, for $x \in [x_{min}, x_{max}]$, wherein $H/h(x)$ is the image scaling ratio of the size $H$ of a physical object, placed at the distance $x$ from the camera, to the size $h$ of its focused image captured by the camera

said method comprising the following steps:

(m1) directing the camera towards the subject's eye and recording a single focused image or a plurality of focused images of the eye, while reading out corresponding focusing element position(s) $z_0$;

(m2) calculating corresponding camera-to-eye distance(s) $x_0$ based on the focusing element position(s) zo using the inverse of the $z(x)$ relation:

$$x_0 = z^{-1}(z_0) \qquad (1)$$

The invention includes also a system for absolute measurement of diameter of a part of an eye or of a distance between a visible light camera and a subject's eye, comprising the visible light camera with adjustable focus and a computational unit, configured and programmed for performing the inventive methods.

The key idea of the invention is to find the $z(x)$ relation, so as to apply the inverse of $z(x)$ to measure desired distance or diameter in absolute units.

## Advantageous effects of the invention

The invention according to the present patent specification has numerous advantages comparing to the known solutions in the prior art. The invented method requires specific preliminary steps for the calibration of the unknown imaging system (that contains plurality of unknown lenses) in visible light mobile camera with adjustable focus. The method can be afterwards implemented without any auxiliary tools for fixing the eye-to-camera distance and without any auxiliary objects (there is no other additional physical or software requirements to implement the inventive method) put in the field of view adjacent to subject's eye providing a size reference.

The method according to the invention does not assume the average statistical diameter of human eye, pupil and/or iris. The method according to the invention provides absolute size measurement of a pupil and/or iris of a human eye observed in visible light expressed in physical units and thus does not require infrared emitter and detector.

The advantage of the described method is also the usage of the unknown in a sense of physical construction (e.g., due to secret know-how or patent protection) mobile camera with adjustable focus for the purpose of precise pupillometric measurements. Thus, any mobile device equipped with visible light camera with adjustable focus, can be used for the implementation of the method offering affordable and reliable measurements of the human eye pupil and/or iris diameter without the pre-existing knowledge about parameters such as: (i) distance between eye and a mobile camera; (ii) magnification of the optical imaging system in the camera; (iii) pixel size of the light sensor in the camera; (iv) the relation between focusing element position and the focusing distance.

## Possible areas of application of the invention

The present invention finds a use primary in a broad field of pupillometry. A pupil and/or iris size determination is nowadays studied within the context of other technological fields, such as medicine, automotive, virtual reality, gaming, education, quick drug detection in law enforcement etc. As the pupil diameter is controlled by the autonomous nervous system via the iris sphincter muscle, the method for dynamic absolute measurement of pupil and/or iris diameter using an unknown visible light mobile camera with adjustable focus, can also find an application in ophthalmology and neurological studies.

## Preferred embodiments of the invention

Now, the invention will be presented in more detail, in a preferred embodiment, with reference to the attached drawing in which:

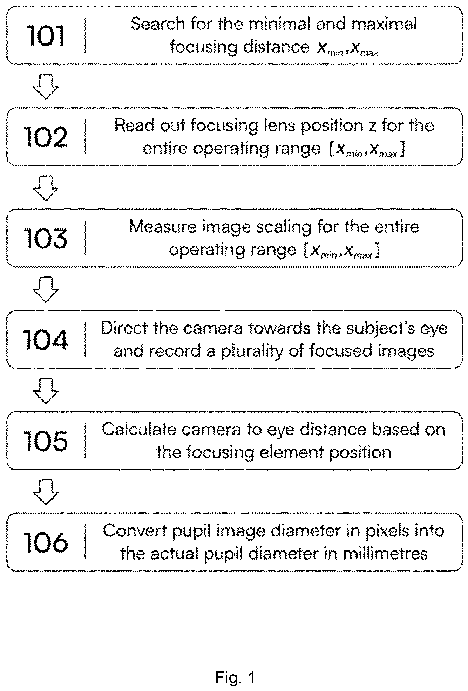

Fig. 1 shows a general overview of method steps including: Searching for the minimal and maximal focusing distance $x_{min}$ and $x_{max}$ (101); Reading out focusing element position z for the entire operating range [$x_{min}$, $x_{max}$] (102); Measuring image scaling for the entire operating range [$x_{min}$, $x_{max}$] (103); Directing the camera towards the subject's eye and record a plurality of focused images (104); Calculating camera to eye distance based on the focusing element position (105); Translating pupil/iris image diameter in pixels into the actual pupil/iris diameter in millimetres (106).

Fig. 2 shows an embodiment of the invention where the unknown visible light mobile camera with adjustable focus is a digital camera (201) and the optical target (202) is a Siemens star. The search for the minimal and maximal focusing distance $x_{min}$ and $x_{max}$ is performed by observing the focused image of the optical target on the light sensor (203), while positioning the optical target (202) as close as possible and as far as possible from the camera imaging system (204). Moving the target outside this range (too close or too far from the camera imaging system) will result in a blurred image (206). Afterwards the optical target is moved along the optical axis (drawn as a dashed line) of the camera imaging system (204) with focused image of the optical target on the light sensor (203) maintained and the position z of the focusing element (205) is retrieved for the entire operating range of focusing distance $x \in [x_{min}, x_{max}]$.

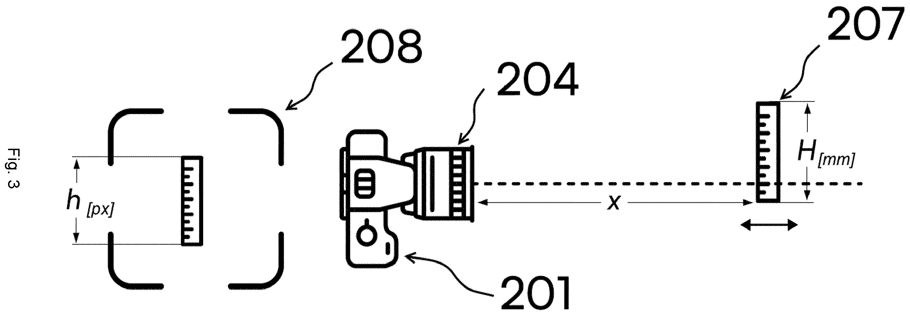

Fig. 3 shows an embodiment of the invention where the unknown visible light mobile camera with adjustable focus is a digital camera (201) and the scaling object (207) is a ruler. The scaling object is moved along the optical axis (drawn as a dashed line) of the camera imaging system (204) and the focused image scaling $H/h$ is measured for the entire operating range of focusing distance $x \in [x_{min}, x_{max}]$.

Fig. 4 shows an embodiment of the invention where the unknown visible light mobile camera with adjustable focus is a digital camera (201). The camera is directed towards the subject's eye (209) and records a plurality of focused images (210), while reading out focusing element position zo (211) within the camera imaging system (204).

Fig. 5 shows an embodiment of the invention where the unknown visible light mobile camera with adjustable focus is a part of a smartphone (301) and the optical target (202) is a Siemens star. The search for the minimal and maximal focusing distance $x_{min}$ and $x_{max}$ is performed by observing the focused image of the optical target on the light sensor (303), while positioning the optical target (202) as close as possible and as far as possible from the camera imaging system (304). Moving the target outside this range (too close or too far from the camera imaging system) will result in a blurred image (306). Afterwards the optical target is moved along the optical axis (drawn as a dashed line) of the camera imaging system (304) with focused image of the optical target on the light sensor (303) maintained and the position z of the focusing element (305) is retrieved for the entire operating range of focusing distance $x \in [x_{min}, x_{max}]$.

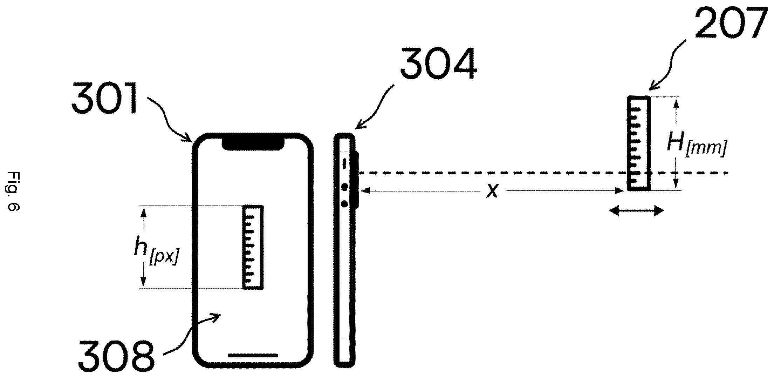

Fig. 6 shows an embodiment of the invention where the unknown visible light mobile camera with adjustable focus is a part of a smartphone (301) and the scaling object (207) is a ruler. The scaling object is moved along the optical axis (drawn as a dotted line) of the camera imaging system (304) and the focus image scaling H/h is measured for the entire operating range of focusing distance $x \in [x_{min}, x_{max}]$.

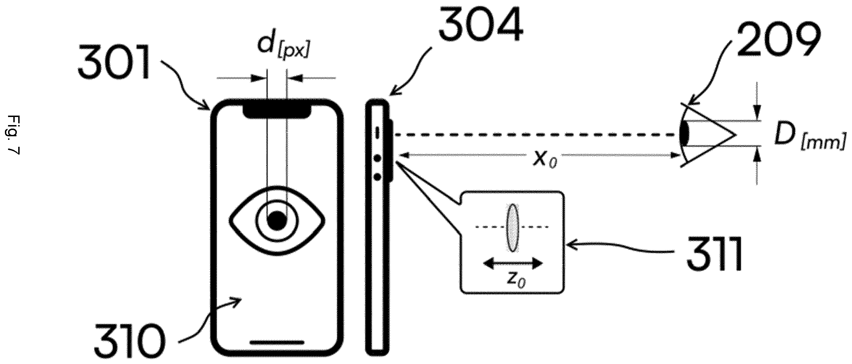

Fig. 7 shows an embodiment of the invention where the unknown visible light mobile camera with adjustable focus is a part of a smartphone (201). The camera is directed towards the subject's eye (209) and records a plurality of focused images (310), while reading out focusing element position $z_0$ (311) within the camera imaging system (304).

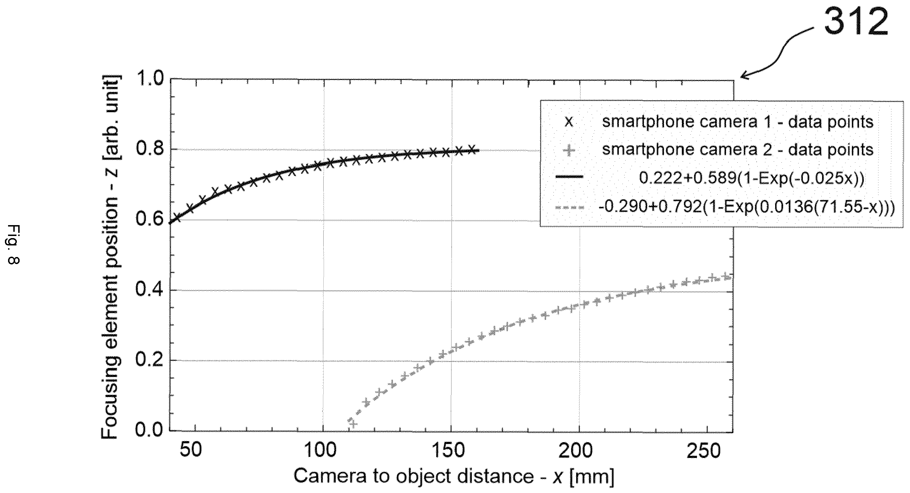

Fig. 8 shows experimentally measured z(x) relation calibration curve (312). In embodiment 2 of the invention using a smartphone camera the z(x) (312) relation is saved e.g., in a form of an interpolated curve, a fitted analytical curve or a lookup table.

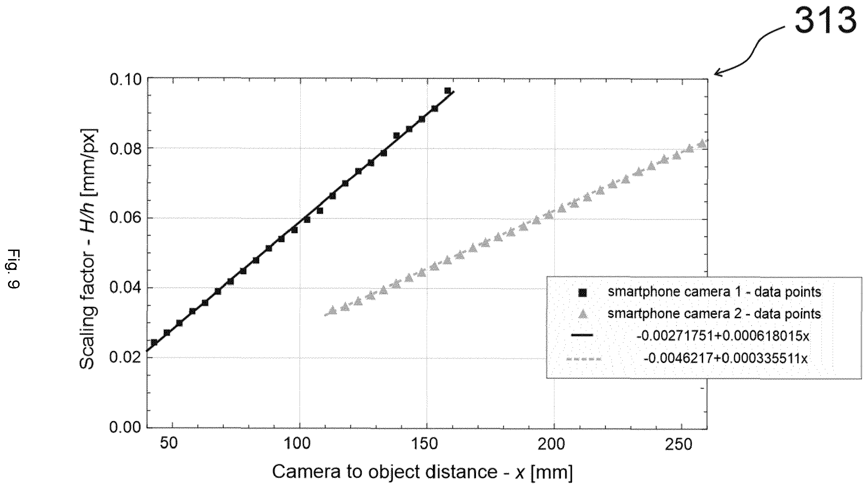

Fig. 9 shows experimentally measured image scaling factor *Hlh(x)* (313). In embodiment 2 of the invention using a smartphone the image scaling factor *Hlh(x)* (313) is saved e.g., in a form of an interpolated curve, a fitted analytical curve or a lookup table.

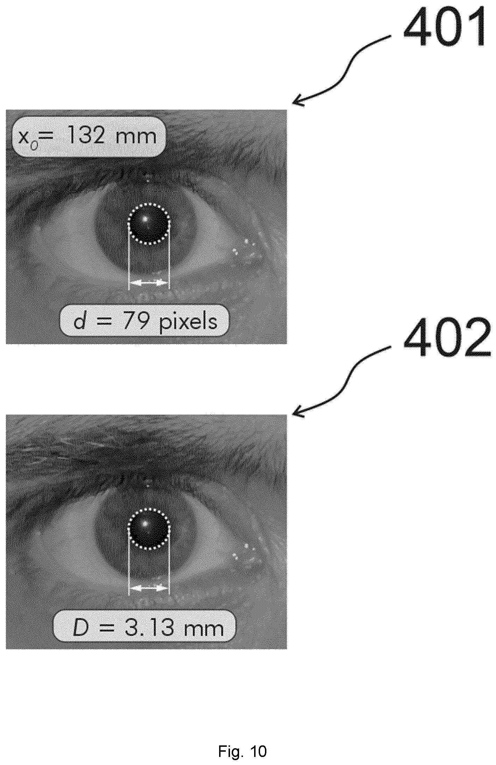

Fig. 10 shows experimental measurement of pupil diameter in accordance to the disclosed method. The eye image has been captured with the calculated camera to eye distance $x_0$= 132 mm. The measured pupil image diameter d = 79 pixels (401) has been converted into actual pupil diameter using the scaling factor (313) and resulted in D = 3.13 mm (402).

The figure uses notation that is consistent with the glossary definitions.

**Embodiments of the invention**

The following examples are included only to illustrate the invention and to explain particular aspects thereof, and not to limit it, and should not be interpreted as the entire range thereof which is defined in the appended patent claims. In the following embodiments, unless indicated otherwise, standard materials and methods used in the technical field were used or manufacturers' recommendations for specific devices, materials and methods were followed. It must be noted that, as used in the described embodiments and the appended claims, the singular forms "a," "an," and "the" include plural referents unless the context clearly dictates otherwise.

**EMBODIMENT 1**

**Absolute measurement of pupil/iris diameter using a generic digital camera**

An absolute measurement of pupil and/or iris diameter using an unknown digital camera (201) with adjustable focus can be realized according to the proposed method consisting of steps presented in fig. 1.
In the first step (101) a search for the minimal and maximal focusing distance $x_{min}$ and $x_{max}$ of the camera has to be performed. An exemplary realization of this step has been presented in fig. 2 point (a). The minimal and maximal focusing distance can be determined by observing the focused image of the optical target on the light sensor (203), while positioning the optical target (202) as close as possible and as far as possible from the camera imaging system (204). Moving the target outside this range (too close or too far from the camera imaging system) will result in a blurred image (206). An alternative realisation for this step is to set the focusing element (205) consecutively in the first and the second extreme position and afterwards for each setting, to find the position of the optical target resulting in sharp image on the light sensor. As the result, the minimal and maximal focusing distance $x_{min}$ and $x_{max}$ of the camera are established. Values of $x_{min}$ and $x_{max}$ can be expressed in any convenient units of distance e.g., in millimetres, mm.

The second step of the method (102) has been illustrated in fig. 2 point (b). In this step the optical target is placed at a varying distance $x$ from the camera and moved along the optical axis (presented as a dashed line) of the camera imaging system (204) while the focusing element position z (205) is either set by the built-in camera algorithm (autofocus) or adjusted manually to ensure that the image of the optical target on the light sensor (203) is always focused. It is critical to maintain a focused image during the scan and avoid focusing element misalignment which result in a blurred image (206). The position z of the focusing element (205) is retrieved for the entire operating range of focusing distance $x \in [x_{min}, x_{max}]$ and the $z(x)$ relation is saved e.g., in a form of an interpolated curve, a fitted analytical curve or a lookup table.

In the third step of the method (103) illustrated in fig. 3, the scaling object of a size H is placed at a varying distance $x$ from the camera and moved along the optical axis (presented as a dotted line) of the camera imaging system (204) and the image of the scaling object of a size h is monitored on the light sensor (208). The values of $H$ and $h$ can be expressed in any convenient units. Typically, $H$ is expressed in millimetres, mm, while $h$ is expressed in pixels (px), as the value in px can be obtained directly from a digital image (e.g., from a *RAVV file*). However, expressing $h$ in other units, in particular - in physical units of distance, such as millimetres, mm, is also possible. The focused image scaling $H/h(x)$ is measured for the entire operating range of focusing distance $x \in [x_{min}, x_{max}]$. For image scaling calibration any object equipped with dimension scale such as a ruler, graph paper or tape measure as well as any object which size is known such as a specific coin, ID card or matchbox can be used. The scaling factor $H/h(x)$ is saved e.g., in a form of an interpolated curve, a fitted analytical curve or a lookup table.

Once the calibration steps are done, the actual measurement of the subject's eye can be performed (104) as illustrated in fig. 4. Before the measurement the camera is directed towards the subject's eye (209) such that the focused image of the pupil/iris (210) occupies the central position of the light sensor. During the measurement a single focused image or a plurality of focused images is recorded and the focusing element position zo (211) is read out and pupil/iris image diameter d is measured, for example in pixels, px. The focusing element position is then used to calculate camera to eye distance $x_0$ with the help of previously recovered z(x) relation (105):

$$z(x_0)=z_0 \rightarrow$$

$$x_0=z^{-1}(z_0) \qquad (1)$$

After the determination of camera to eye distance the pupil/iris image diameter d is converted into actual diameter of a pupil/iris D [mm] with a use of the scaling factor $H/h(x)$:

$$D/d=H/h(x_0) \rightarrow$$

$$D=d*H/h(x_0) \qquad (2)$$

It must be noted that the relations (1) and (2) presented above are essential relations used by the present inventive method. Therefore, they hold true not only for the embodiment discussed here, but for any embodiment of the present invention.

Moreover, it must be noted, not only for the purpose of the embodiment discussed here, but for any embodiment of the present invention, that as an alternative to the first, second and third steps above (and generally speaking, as an alternative to calibration steps leading to establishing $x_{min}$, $x_{max}$, $z(x)$ and $H/h(x)$ at least for x in the range $[x_{min}, x_{max}]$) - the characteristics of the camera comprising $x_{min}$, $x_{max}$, $z(x)$ and $H/h(x)$ in any usable form can be provided prior to the measurements of the subject's eye. For example, relevant data can be provided by a third party, such as the camera's manufacturer, for example in the form of an interpolated curve, a fitted analytical curve or a lookup table. Such characteristics, once provided, is used in the calculation steps of the present inventive method.

In the presented method it is beneficial to use a camera with low f-number imaging system such as f/1 or f/1.4.

Low f-number results in shallow depth-of-field which helps to increase the precision of optical target positioning along the optical axis in the method steps (102) and (103). Similarly, during the actual measurement (104) of subject's eye, shallow depth-of-field reduces the uncertainty of focusing element position $z_0$ and in consequence increases the calculation precision of camera to eye distance $x_0$. By the same token in the presented method, it is beneficial to perform the actual measurement (104) with camera to eye distance close to the smallest value of focusing distance operating range [$x_{min}$, in order to additionally reduce the depth-of-field.

It must be noted that in the described method, the optical target (202) and the scaling object (207) might be the same object.

## EMBODIMENT 2

### Absolute measurement of pupil/iris diameter using a smartphone

An absolute measurement of pupil and/or iris diameter using an unknown digital camera, where the unknown visible light mobile camera with adjustable focus is a part of a smartphone (301) with adjustable focus can be realized according to the proposed method consisting of steps presented in fig. 1. In this particular embodiment Apple iPhone 13 Pro smartphone was used.

In the first step (101) a search for the minimal and maximal focusing distance $x_{min}$ and $x_{max}$ of the camera has to be performed. An exemplary realization of this step has been presented in fig. 5 point (a). The minimal and maximal focusing distance can be determined by observing the focused image of the optical target on the light sensor (303), while positioning the optical target (202) as close as possible and as far as possible from the camera imaging system (304). Moving the target outside this range (too close or too far from the camera imaging system) will result in a blurred image (306). An alternative realisation for this step is to set the focusing element (305) consecutively in the first and the second extreme position and afterwards for each setting, to find the position of the optical target resulting in sharp image on the light sensor. The step results in a pair of retrieved parameters $x_{min}$ = 112 mm, and $x_{max}$ = 260 mm.

The second step of the method (102) has been illustrated in fig. 5 point (b). In this step the optical target is moved along the optical axis $x$ (presented as a dashed line) of the camera imaging system (304) while the focusing element position $z$ (305) is either set by the built-in camera algorithm (autofocus) or adjusted manually to ensure that the image of the optical target on the light sensor (303) is always focused. It is critical to maintain a focused image during the scan and avoid focusing element misalignment which result in a blurred image (306). The position z of the focusing element (305) is retrieved for the entire operating range of focusing distance $x \in$ [$x_{min}$, $x_{max}$] and the z(x) relation (312) is saved e.g., in a form of an interpolated curve, a fitted analytical curve or a lookup table.

In the next step of the method (103) illustrated in fig. 6, the scaling object of a size $H$ [mm] is moved along the optical axis (presented as a dashed line) of the camera imaging system (304) and the image of the scaling object of a size $h$ is monitored on the light sensor (308). The focused image scaling $H/h(x)$ (313) is measured for the entire operating range of focusing distance $x \in$ [$x_{min}$, $x_{max}$]. For image scaling calibration any object equipped with dimension scale such as a ruler, graph paper or tape measure as well as any object which size is known such as a specific coin, ID card or matchbox can be used. The scaling factor $Hlh(x)$ (313) is saved e.g., in a form of an interpolated curve, a fitted analytical curve or a lookup table.

Once the calibration steps are done, the actual measurement of the subject's eye can be performed (104) as illustrated in fig. 7. Before the measurement the camera is directed towards the subject's eye (209) such that the focused image of the pupil/iris (310) occupies the central position of the light sensor. During the measurement a plurality of focused images is recorded and the focusing element position $z_0$ (311) is read out and pupil and/or iris image diameter $d$ [mm] is measured. The focusing element position is then used to calculate camera to eye distance $x_0$ with the help of previously recovered $z(x)$ (312) in relation to (105). After the determination of camera to eye distance the pupil/iris image diameter $d$ [px] is converted into actual diameter of a pupil and/or iris $D$ [mm] with a use of the scaling factor $Hlh(x)$ (313). The final result of the method has been presented in fig. 10, where eye image has been collected with the calculated camera to eye distance $x_0$= 132 mm. The measured pupil image diameter $d$ = 79 pixels (401) has been converted into actual pupil diameter using the scaling factor (313) and resulted in D = 3.13 mm (402).

In the presented method it is beneficial to use a camera with low f-number imaging system such as f/1 or f/1.4. Low f-number results in shallow depth-of-field which helps to increase the precision of optical target positioning along the optical axis in the method steps (102) and (103). Similarly, during the actual measurement (104) of subject's eye, shallow depth-of-field reduces the uncertainty of focusing element position $z_0$ and in consequence increases the calculation precision of camera to eye distance $x_0$. By the same token in the presented method, it is beneficial to perform the actual measurement (104) with camera to eye distance close to the smallest value of focusing distance operating range [$x_{min}$, $x_{max}$] in order to additionally reduce the depth-of-field. In this particular embodiment the experimentally measured uncertainty of $x$ determination equals 1.2 mm, for $x$ close to $x_{min}$, and 3.6 mm for $x$ close to $x_{max}$. The uncertainty of $x$ determination is defined as a distance range where focusing element position does not change despite of the optical target movement along the optical axis.

It must be noted that in the described method, the optical target (202) and the scaling object (207) might be the same object.

**EMBODIMENT 3**

**Absolute measurement of pupil/iris diameter using a smartphone equipped with LIDAR**

An absolute measurement of pupil and/or iris diameter using an unknown digital camera, where the unknown visible light mobile camera with adjustable focus is a part of a smartphone (301) with adjustable focus, wherein a smartphone comprising a LIDAR device. In this particular embodiment Apple iPhone 13 Pro smartphone was used. LIDAR is a device for determining ranges (variable distance) by targeting an eye or a surface with a laser (visible light or IR) and measuring the time for the reflected light to return to the receiver.

In this particular embodiment, the position z of the focusing element (305) is retrieved for the entire operating range of focusing distance $x \in [x_{min}, x_{max}]$ and the z(x) is determined using LIDAR device of a smartphone.

The actual measurement of the subject's eye can be performed (104) as illustrated in fig. 7. Before the measurement the camera is directed towards the subject's eye (209) such that the focused image of the pupil/iris (310) occupies the central position of the light sensor. During the measurement a plurality of focused images is recorded and the focusing element position zo (311) is read out and pupil and/or iris image diameter $d$ [mm] is measured. The focusing element position is then used to calculate camera to eye distance $x_0$ with the help of previously recovered $z(x)$ in relation to (105). After the determination of camera to eye distance the pupil/iris image diameter $d$ [px] is converted into actual diameter of a pupil and/or iris $D$ [mm] with a use of the scaling factor $H/h(x)$.

It must be noted that in the described method, the optical target (202) and the scaling object (207) is the same object.

**SUMMARY OF EMBODIMENTS**

In summary, numerous modifications and variations of the present disclosure are possible in light of the above-described measurement method. It is therefore to be understood that within the scope of the appended claims, the embodiment may be practiced otherwise than as specifically described herein. For example, advantageous results may be achieved if the steps of the disclosed techniques were performed in a different sequence, if components in the disclosed systems were combined in a different manner, or if the components were replaced or supplemented by other components. The functions, processes, and algorithms described herein may be performed in hardware or software (computer-implemented invention) executed by hardware (smartphone or other device containing a camera with an adjustable focus), including computer processors and/or programmable processing circuits configured to execute program code and/or computer instructions to execute the functions, processes, and algorithms described herein. A processing circuit includes a programmed processor, as a processor includes circuitry. A processing circuit also includes devices such as an application specific integrated circuit (ASIC) and conventional circuit components arranged to perform the recited functions. The claimed features and associated function (technical effects) described herein may also be executed by various distributed components of a system. For example, one or more processors may execute described method.

It must be noted that the relations (1) and (2) presented above are essential relations used by the present inventive method.

Moreover, it must be noted, that as an alternative to calibration steps leading to establishing $x_{min}$, $x_{max}$, $z(x)$ and $H/h(x)$ at least for $x$ in the range $[x_{min}, x_{max}]$ - the characteristics of the camera comprising $x_{min}$, $x_{max}$, $z(x)$ and $H/h(x)$ in any usable form can be provided prior to the measurements of the subject's eye. For example, relevant data can be provided by a third party, such as the camera's manufacturer, for example in the form of an interpolated curve, a fitted analytical curve or a lookup table. Such characteristics, once provided, is used in the calculation steps of the present inventive method.

The focused image scaling $H/h(x)$ can also be measured over any smaller range of $x \in [x_{low}, x_{high}]$, as long as it covers the expected distances of the ultimate eye measurements. This means in practice that $x_{min} \leq x_{low} < x_{high} \leq x_{max}$. Most preferably $x_{min} = x_{low}$ while $x_{high} = x_{max}$.

While certain embodiments have been described, these embodiments have been presented by way of example only, and are not intended to limit the scope of the inventions. Indeed, the novel methods and computer implemented method and device to perform the described herein can be embodied in a variety of other forms; furthermore, various omissions, substitutions and changes in the form of the methods, devices and computer implemented method described herein can be made without departing from the scope of the inventions. The accompanying claims are intended to cover such forms or modifications as would fall within the scope of the inventions.

**Claims**

1.  A method for absolute measurement of diameter of a part of an eye, using a visible light camera (201, 301) with adjustable focus, said camera having the following camera characteristics:

    - the minimal and maximal focusing camera-to-object distance $x_{min}$ and $x_{max}$ of the camera imaging system;
    - the z(x) relation, for $x \in [x_{min}, x_{max}]$; wherein $z(x)$ is the position of the focusing element (205, 305) of the camera (201, 301) which results in a focused image on an object located at the distance $x$ from the camera;
    - the *Hlh(x)* relation, for $x \in [x_{min}, x_{max}]$; wherein *H/h(x)* is the image scaling ratio of the size *H* of a physical object, placed at the distance $x$ from the camera (201, 301), to the size h of its focused image (208, 308) captured by the camera (201, 301)

    said method comprising the following steps:

    (m1) directing the camera (201, 301) towards the subject's eye (209) and recording a single focused image or a plurality of focused images (210, 310) of the part of the subject's eye, while reading out corresponding focusing element position(s) zo (211, 311) and measuring corresponding diameter(s) *d* of the focused image(s) of the part of the eye;
    (m2) calculating corresponding camera-to-eye distance(s) $x_0$ based on the focusing element position(s) zo using the inverse of the *z(x)* relation:

    $$x_0 = z^{-1}(z_0) \qquad (1)$$

    (m3) converting the diameter(s) d of one or more images of the part of the eye obtained in step (m1) into actual diameter(s) *D* of the part of the eye for each focused image collected in step (m1), using the *Hlh(x)* relation and the corresponding value(s) of $x_0$ obtained in step (m2):

    $$D = d * H/h(x_0) \qquad (2)$$

2.  The method according to claim 1, wherein said camera characteristics is provided by a third party, such as the camera's (201, 301) manufacturer, preferably in the form of an interpolated curve, a fitted analytical curve or a lookup table.

3.  The method according to claims 1 or 2, wherein said camera characteristics is established in a procedure comprising the following steps:

    (c1) establishing the minimal and maximal focusing camera-to-object distance $x_{min}$ and $x_{max}$ of the camera imaging system;
    (c2) reading out focusing element position (205, 305) $z(x)$ resulting in a focused image (203, 303) of an optical target (202) located at the distance $x$ from the camera, for at least some $x \in [x_{min}, x_{max}]$, preferably for the entire operating range of the focusing distance $[x_{min}, x_{max}]$, by means of the optical target (202) moved along the optical axis of the camera, thereby obtaining the $z(x)$ relation for $x \in [x_{min}, x_{max}]$;
    (c3) measuring focused image scaling ratio *Hlh(x)* of the physical size H of a scaling object (207), placed at the distance $x$ from the camera (201, 301), to the size h of its focused image (208, 308) obtained in the camera (201, 301), for at least some $x \in [x_{min}, x_{max}]$, preferably for the entire operating range of the focusing distance $[x_{min}, x_{max}]$, by means of the scaling object (207) moved along the optical axis of the camera, thereby obtaining the *Hlh(x)* relation for $x \in [x_{min}, x_{max}]$.

4.  The method according to claim 3, wherein in steps (c1) and/or (c2) the focusing element position (205, 305) resulting in the focused image is set using a built-in algorithm implemented in the camera (201, 301).

5.  The method according to claim 3 or 4, wherein in steps (c1) and/or (c2) the focusing element position (205, 305) resulting in the focused image is set manually by a user.

6.  The method according to any one of the preceding claims, wherein the camera (201, 301) is a digital camera.

7. The method according to any one of the preceding claims, wherein the camera (201, 301) is a camera with adjustable focus being a part of a mobile electronic device, in particular is a built-in camera of a smartphone or a tablet

8. The method according to claim 7, wherein the mobile electronic device contains a single camera or a plurality of cameras.

9. The method according to any one of the preceding claims, wherein the camera (201, 301) is a camera with adjustable focus which contains a plurality of lenses.

10. The method according to any one of the preceding claims, wherein the camera (201, 301) contains an imaging system with f-number in a range of f/0.95-f/2.8.

11. The method according to any one of the preceding claims, wherein the distance between camera imaging system (204, 304) and the subject's eye (209) is more than 1.5 cm and less than 75 cm, preferably more than 2 cm and less than 50 cm, more preferably more than 3 cm and less than 40 cm, still more preferably more than 4 cm and less than 30 cm.

12. The method according to any one of the preceding claims, wherein the part of an eye is a pupil or an iris.

13. The method according to any one of the preceding claims, **characterized in that** it is computer-implemented.

14. A method for absolute measurement of a distance between a visible light camera (201, 301) and a subject's eye (209), wherein the camera (201, 301) has adjustable focus and the following camera characteristics:

   - the minimal and maximal focusing camera-to-object distance $x_{min}$ and $x_{max}$ of the camera imaging system;
   - the $z(x)$ relation, for $x \in [x_{min}, x_{max}]$, wherein $z(x)$ is the position of the focusing element (205) of the camera (201, 301) which results in a focused image on an object located at the distance $x$ from the camera;
   - the $H/h(x)$ relation, for $x \in [x_{min}, x_{max}]$, wherein $H/h(x)$ is the image scaling ratio of the size $H$ of a physical object, placed at the distance $x$ from the camera (201, 301), to the size $h$ of its focused image (208, 308) captured by the camera (201, 301)

   said method comprising the following steps:

   (d1) directing the camera (201, 301) towards the subject's eye (209) and recording a single focused image or a plurality of focused images (210, 310) of the eye (209), while reading out corresponding focusing element position(s) zo (211, 311);
   (d2) calculating corresponding camera-to-eye distance(s) $x_0$ based on the focusing element position(s) zo using the inverse of the $z(x)$ relation:

$$x_0 = z^{-1}(z_0) \qquad\qquad (1)$$

15. A system for absolute measurement of diameter of a part of an eye or of a distance between a visible light camera (201, 301) and a subject's eye (209), comprising the visible light camera (201, 301) with adjustable focus and a computational unit, configured and programmed for performing the method according to any one of the preceding claims.

| 101 | Search for the minimal and maximal focusing distance $x_{min}, x_{max}$ |
|---|---|

⇩

| 102 | Read out focusing lens position z for the entire operating range $[x_{min}, x_{max}]$ |
|---|---|

⇩

| 103 | Measure image scaling for the entire operating range $[x_{min}, x_{max}]$ |
|---|---|

⇩

| 104 | Direct the camera towards the subject's eye and record a plurality of focused images |
|---|---|

⇩

| 105 | Calculate camera to eye distance based on the focusing element position |
|---|---|

⇩

| 106 | Convert pupil image diameter in pixels into the actual pupil diameter in millimetres |
|---|---|

Fig. 1

Fig. 2

Fig. 3

Fig. 4

Fig. 5

Fig. 6

Fig. 7

Fig. 8

Fig. 9

Fig. 10

Europäisches
Patentamt

European
Patent Office

Office européen
des brevets

# EUROPEAN SEARCH REPORT

**Application Number**

EP 22 46 1644

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/084203 A1 (HONJO KENICHI [JP] ET AL) 22 March 2018 (2018-03-22) | 1,2,6-15 | INV.<br>A61B3/11 |
| A | * paragraphs [0021] – [0030], [0038], [0041] * | 3-5 | G06F21/32<br>G06V40/19 |
| | ----- | | G02C13/00 |
| A | US 2005/264758 A1 (WAKAMORI MASAHIRO [JP]) 1 December 2005 (2005-12-01) | 1-15 | |
| | * paragraphs [0088] – [0101] * | | |
| | ----- | | |

**TECHNICAL FIELDS SEARCHED (IPC)**

A61B
G06F
G06T
G06V
G02C

The present search report has been drawn up for all claims

| Place of search | Date of completion of the search | Examiner |
|---|---|---|
| Munich | 11 May 2023 | Martelli, Luca |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 46 1644

This annex lists the patent family members relating to the patent documents cited in the above-mentioned European search report.
The members are as contained in the European Patent Office EDP file on
The European Patent Office is in no way liable for these particulars which are merely given for the purpose of information.

11-05-2023

| Patent document cited in search report | Publication date | Patent family member(s) | Publication date |
|---|---|---|---|
| US 2018084203 A1 | 22-03-2018 | JP 6621027 B2 | 18-12-2019 |
| | | JP 2018046535 A | 22-03-2018 |
| | | US 2018084203 A1 | 22-03-2018 |
| US 2005264758 A1 | 01-12-2005 | CN 1819796 A | 16-08-2006 |
| | | EP 1690494 A1 | 16-08-2006 |
| | | JP 3781028 B2 | 31-05-2006 |
| | | JP 2005103122 A | 21-04-2005 |
| | | US 2005264758 A1 | 01-12-2005 |
| | | WO 2005032371 A1 | 14-04-2005 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018222897 A1
- US 10034605 B2
- WO 2014163891 A1
- WO 2020168009 A1
- WO 2021211886 A1
- US 11122972 B2
- US 9720259 B2
- US 20170347878 A1